# EUROPEAN PATENT SPECIFICATION

(11) **EP 0 785 773 B1**
(45) Date of publication and mention of the grant of the patent: **03.01.2001**
(21) Application number: 95935710.4
(22) Date of filing: 12.10.1995
(51) Int. Cl.: A61K 9/127, A61K 31/685

(54) **ETHER LIPID LIPOSOMES AND THEIR THERAPEUTIC USE**
ETHERLIPID-LIPOSOMEN UND DEREN THERAPEUTISCHE VERWENDUNG
LIPOSOMES COMPORTANT DES LIPIDES ETHERS ET LEUR UTILISATION THERAPEUTIQUE

(30) Priority: 14.10.1994 US 323042
(43) Date of publication of application: 30.07.1997
(73) Proprietor: THE LIPOSOME COMPANY, INC., Princeton, NJ 08540 (US)
(72) Inventor: MAYHEW, Eric, Monmouth Junction, NJ 08852 (US); JANOFF, Andrew, S., Yardley, PA 19067 (US); AHMAD, Imran, Plainsboro, NJ 08536 (US); BHATIA, Suresh, K., New Delhi 110019 (IN)
(74) Representative: Weiss, Wolfgang, Dipl.-Chem. Dr.
(86) International application number: PCT/US95/12721
(87) International publication number: WO 96/11670

(56) References cited:
- EP-A- 0 213 523
- WO-A-94/27580
- DE-A- 4 132 345
- DE-C- 4 408 011

## Description

This invention is directed to ether lipid liposomes, and to their therapeutic use, for example, in the treatment of cancers.

Ether lipids are synthetic analogues of platelet activating factor (PAF; 1-O-alkyl-2-acetyl-sn-glycero-3-phosphocholine), an effector generally believed to be involved in a variety of physiological processes, such as inflammation, the immune response, allergic reactions and reproduction. Ether lipids have been shown to be effective antitumor agents in animals, and are believed to be selectively cytotoxic to a broad variety of cancer cells (see, for example, Dietzfelbinger et al. (1993); Zeisig et al. (1993); Powis et al. (1990); Berdel (1991); Bhatia and Hadju (1991); Reed et al. (1991): Workman (1991); Workman et al. (1991); Bazill and Dexter (1990); Berdel (1990); Counsell et al. (1990); Tritton and Hickman (1990); Muschiol et al. (1990); Layton et al. (1980); Runge et al. (1980); Great Britain Patent No. 1,583,661; U.S. Patent No. 3,752,886). Ether lipids have also been shown to be antimetastatic and anti-invasive, and to be capable of cell differentiation induction. None of these disclosures teach liposomal ether lipids with the advantageous properties of this invention's liposomal formulations. Several ether lipids are currently the subject of clinical trial study.

Mechanisms of ether lipid cytotoxicity, while not definitively established, appear to involve action at, and possible disruption of, the cell membrane. The selective cytotoxicity of ether lipids may involve intracellular accumulation and differential activity of alkyl cleavage enzymes. Ether lipids may also be selective inhibitors of phosphatidylinositol phospholipase C and protein kinase C activities, as well as of phosphatidylcholine biosynthesis.

Ether lipids can, in addition to their antitumor activity, be hemolytic, that is they can lyse red blood cells. Furthermore, clinical trial results show that ether lipid administration can lead to hepatic dysfunction and gastrointestinal disorders. Applicants have found that certain liposomal formulations of ether lipids can buffer these toxicities.

### SUMMARY OF THE INVENTION

A liposome having a lipid bilayer comprising a headgroup-derivatized lipid and an ether lipid is provided herein. Preferably, the liposome has a diameter of less than about 200 nm, more preferably, from greater than about 50 nm to less than about 200 nm.

The headgroup-derivatized lipid is preferably a phosphatidylethanolamine-dicarboxylic acid. The dicarboxylic acid, for example, glutaric, sebacic, succinic, or tartaric acid, is preferably glutaric acid. The phosphatidylethanolamine (PE) is preferably dipalmitoylphosphatidylethanolamine (DPPE), palmitoyloleoyl phosphatidylethanolamine (POPE) or dioleoyl phosphatidylethanolamine (DOPE). More preferably, the headgroup-derivatized lipid is DOPE-GA. The headgroup-derivatized lipid can, but is not required to, be a circulation-enhancing lipid.

The ether lipid has the chemical formula: 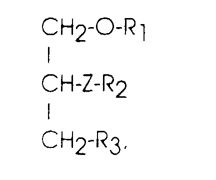 wherein R₁ is Y₁Y₂, Y₂ is CH₃ or CO₂H and Y₁ is (CH₂)_{n 1} (CH=CH)ₙ₂(CH₂)ₙ₃(CH=CH)ₙ₄(CH₂)ₙ₅(CH=CH)ₙ₆(CH₂)ₙ₇ (CH=CH)ₙ₈(CH₂)ₙ₉. n1 is equal to zero or an integer of from 3 to 23; n3 is equal to zero or an integer of from 1 to 21; n5 is equal to zero or an integer of from 1 to 18; n7 is equal to zero or an integer of from 1 to 15; n9 is equal to zero or an integer of from 1 to 12; and each of n2, n4, n6 and n8 is independently equal to zero or 1. The sum of n1 +2n2 +n3 +2n4 +n5 +2n6 + n7 +2n8 +n9 is equal to an integer of from 3 to 23. Y₂ is preferably CH₃, and R₁ is preferably (CH₂)ₙ₁CH₃, that is, a saturated hydrocarbon. The preferred saturated hydrocarbon is R₁ is (CH₂)₁₇CH₃.

Z is preferably O, but can also be S. R₂ can also be Y₁Y₂, and can be the same as, or different than, R₁. Preferably, R₂ is (C(X₁)ₙ₁₀(X₂)ₙ₁₁)ₙ₁₂CX₃X₄X₅. Each of X₁, X₂, X₃, X₄, and X₅ is independently hydrogen or fluorine, but is preferably hydrogen. n10 is equal to zero, 1 or 2; n11 is equal to zero, 1, or 2; and n12 is equal to zero or an integer of from 1 to 23, but is preferably, zero. When n12 is zero, R₂ is CX₃X₄X₅, X₃, X₄, and X₅ are preferably H, and R₂ is CH₃. When n12 is not zero, the sum of n10 + n11 is equal to 2, n12 is preferably equal to 1, and R₂ is preferably CH₂CH₃, CH₂CF₃ or CF₂CF₃.

R₃ has the formula R₅-P(O)₂-O-R₆, R₅ is O, S or NH, but is preferably O, and R₆ is CH₂CH₂N(CH₃)₃ (choline) CH₂CH₂NH₂, CH₂CH(OH)CH₂OH, or CH₂CH₂NHC(O)R₇, but is preferably choline. Accordingly, R₃ is preferably -O-P(O)₂-O-CH₂CH₂N(CH₃)₃. R₇ is Y₂CH₃ or Y₂CO₂H.

Accordingly, the preferred ether lipid is: 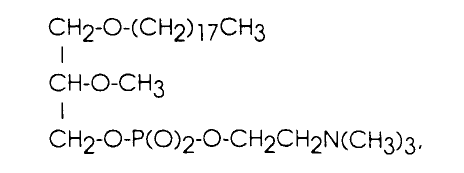 that is, the ether lipid is 1-O-octadecyl-2-O-methyl-sn-glycero-3-phosphocholine ("ET-18-OCH₃").

The liposome of this invention can also comprise a neutral lipid, such as a phosphatidylcholine (PC). Preferably, the PC is egg phosphatidylcholine (EPC), dioleoyl phosphatidylcholine (DOPC) or palmitoyloleoyl phosphatidylcholine (POPC). More preferably, the PC is DOPC. The liposome can also comprise a sterol, preferably, cholesterol. The liposome can also comprise a circulation-enhancing lipid, which can comprise a phosphatidylethanolamine, and a dicarboxylic acid, ganglioside or polyethylene glycol.

In a preferred embodiment of the invention, the liposome has a lipid bilayer comprising an ether lipid, a headgroup-derivatized lipid, a sterol and a neutral lipid. Preferably, the ether lipid is ET-18-OCH₃, the headgroup-derivatized lipid is a phosphatidylethanolamine-dicarboxylic acid, more preferably, DOPE-GA, the sterol is cholesterol and the neutral lipid is a PC, more preferably, DOPC. The bilayer generally comprises from about 10 mole percent to about 30 mole percent, more preferably about 20 mole percent, of the ether lipid, from about 5 mole percent to about 20 mole percent, more preferably about 10 mole percent of the headgroup-derivatized lipid, from about 10 mole percent to about 50 mole percent, more preferably about 30 mole percent of the sterol and from about 10 mole percent to about 50 mole percent, more preferably about 40 mole percent of the neutral lipid. Most preferably, the bilayer comprises about 20 mole percent "ET-18-OCH₃", about 10 mole percent DOPE-GA, about 30 mole percent cholesterol and about 40 mole percent DOPC.

The liposome can comprise an additional bioactive agent, which is preferably an antimicrobial agent, antineoplastic agent, anti-inflammatory agent, therapeutic lipid or hematopoietic cell growth stimulating agent. The liposome can be dehydrated.

Also provided herein is a pharmaceutical composition comprising a pharmaceutically acceptable carrier and the ether lipid liposome of this invention. Further provided is a method of administering an ether lipid to an animal which comprises administering to the animal a pharmaceutical composition comprising a pharmaceutically acceptable carrier and a liposome having a lipid bilayer which comprises an ether lipid and a headgroup-derivatized lipid, the ether lipid having the chemical formula: 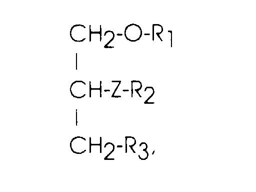 Preferably the animal is a human. Preferably, the liposome has a diameter of less than about 200 nm, more preferably, from greater than about 50 nm to less than about 200 nm.

Preferably, a saturated hydrocarbon, most preferably, (CH₂)₁₇CH₃ is attached at the 1 position of the ether lipid through the O. Preferably, R₂ is (C(X₁)ₙ₁₀(X₂)ₙ₁₁)ₙ₁₂CX₃X₄X₅, more preferably, CH₃. Preferably, the polar group at the 3 position is phosphorylcholine. Accordingly, the ether lipid is preferably 1-O-octadecyl-2-O-methyl-sn-glycero-3-phosphocholine (ET-18-OCH₃).

Preferably, the headgroup-derivatized lipid is a PE-dicarboxylic acid,, most preferably, DOPE-GA. The derivatized lipid can be a circulation-enhancing lipid. Preferably the bilayer comprises cholesterol and a phosphatidylcholine, more preferably, EPC, POPC or DOPC. The liposome can also comprise a circulation-enhancing lipid

In a particularly preferred embodiment of the invention, the hposome comprises 20 mole percent of an ether lipid, preferably ET-18-OCH₃, about 10 mole percent DOPE-GA, about 30 mole percent cholesterol and about 40 mole percent DOPC.

The method of this invention can be used to administer an ether lipid to an animal afflicted with a cancer, including, but not limited to, carcinomas, leukemias, myelomas, neuroblastomas, sarcomas, of the lungs, brain, ovaries, colon or breasts. Such a method generally comprises administering to the animal an amount of the liposome which comprises an antineoplastic effective amount of the ether lipid. Antineoplastic effective amounts of the liposomal ether lipid are generally from about 0.1 mg of the ether lipid per kg of the body weight of the animal treated to about 1000 mg of the ether lipid per kg of body weight. Preferably, the antineoplastic effective amount of the liposomal ether lipid is from about 1 mg of the ether lipid per kg of body weight to about 500 mg per kg. Most preferably, the anticancer effective amount of the ether lipid is 200 mg per kg of body weight. Anticancer effective amounts of a liposomal ether lipid can be administered in multiple administrations. Multiple doses of a liposomal ether lipid, each dose comprising an anticancer effective amount of the ether lipid, can also be administered. Metastases or invasion of the cancer can be inhibited by ether lipid administration.

The method of this invention can also be used to administer an ether lipid to an animal afflicted with an inflammatory disorder, e.g., an arthritic condition, allergic reaction or asthmatic disorder. Such a method generally comprises administering an anti-inflammation effective amount of the liposomal ether lipid to the animal. Anti-inflammation effective amounts of ether lipids are generally from about 0.1 mg of the ether lipid per kg of the body weight of the animal treated to about 1000 mg of the ether lipid per kg of body weight.

The method of this invention can also comprise administration of a biologically active agent in addition to the ether lipid administered. This additional biologically active agent, which can be associated, or unassociated with the liposome, is, for example, but not limited to, an antimicrobial agent, antineoplastic agent, anti-inflammatory agent, therapeutic lipid or hematopoietic cell growth stimulating agent.

### BRIEF DESCRIPTION OF THE DRAWINGS

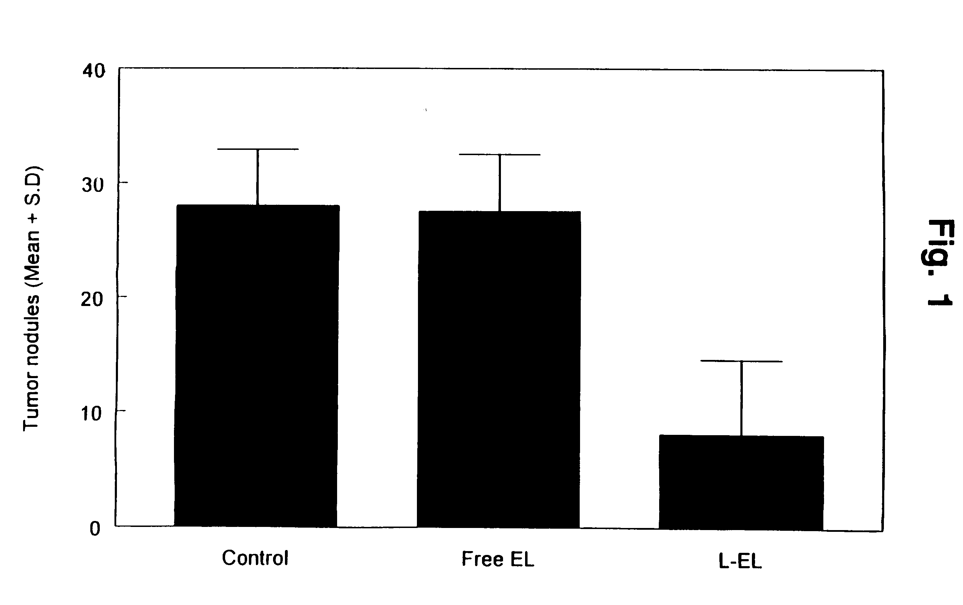

- **FIGURE 1.**: Effects of Liposomal Ether Lipid on Lung Metastasis in Mice. Liposomal formulation: EPC/Cholesterol/DPPE-GA/EL (4:3:1:2). Dose: 25 mg/kg x 5. x-axis: Buffer control, liposome control (no ether lipid), free ether lipid, liposomal ether lipid; y-axis: number of tumor nodules (mean plus s.d.).

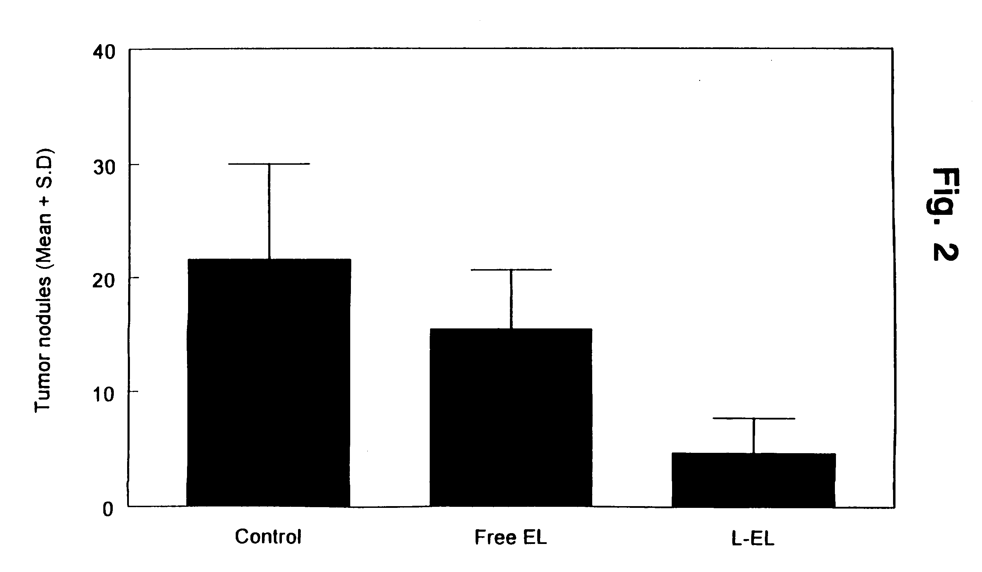

- **FIGURE 2**: Effects of Liposomal Ether Lipid on Lung Metastasis in Mice. Liposome formulation: DOPC/Chol/DOPE-GA/EL (4:3:1:2). Dose: 5 injections, x-axis: Buffer control, liposome control, free ether lipid, liposomal ether lipid (25 mg/kg of body weight dose), liposomal ether lipid (100 mg per kg dose); y-axis: number of tumor nodules (mean plus s. d.).

### DETAILED DESCRIPTION OF THE INVENTION

Provided herein is a liposome having a lipid bilayer comprising a headgroup-derivatized lipid and an ether lipid. Liposomes are self-assembling structures comprising one or more lipid bilayers, each of which surrounds an aqueous compartment and comprises two opposing monolayers of amphipathic lipid molecules. These comprise a polar (hydrophilic) headgroup region covalently linked to one or two non-polar (hydrophobic) acyl chains. Energetically unfavorable contacts between the hydrophobic acyl chains and the aqueous medium are generally believed to induce lipid molecules to rearrange such that the polar headgroups are oriented towards the aqueous medium while the acyl chains reorient towards the interior of the bilayer. An energetically stable structure is formed in which the acyl chains are effectively shielded from coming into contact with the aqueous medium.

Liposomes can be made by a variety of methods (for a review, see, for example, Deamer and Uster (1983)). These methods include without limitation: Bangham's methods for making muiltilamellar liposomes (MLVs); Lenk's, Fountain's and Cullis' methods for making MLVs with substantially equal interlamellar solute distribution (see, for example, U.S. Patent Nos. 4,522,803, 4,588,578, 5,030,453, 5,169,637 and 4,975,282); and Paphadjopoulos et al.'s reverse-phase evaporation method (U.S. Patent No. 4,235,871) for preparing oligolamellar liposomes. Unilamellar vesicles can be produced from MLVs by such methods as sonication (see Paphadjopoulos et al. (1968)) or extrusion (U.S. Patent No. 5,008,050 and U.S. Patent No. 5,059,421). The ether lipid liposome of this invention can be produced by the methods of any of these disclosures, the contents of which are incorporated herein by reference.

Various methodologies, such as sonication, homogenization, French Press application, milling and extrusion can be used to size reduce liposomes, that is to prepare liposomes of a smaller size from larger liposomes. Tangential flow filtration (see W089/008846), can also be used to regularize the size of liposomes, that is, to produce liposomes having a population of liposomes having less size heterogeneity, and a more homogeneous, defined size distribution. The liposome of this invention can be unilamellar or multilamellar, and preferably has a diameter of less than about 200 nm, more preferably, from greater than about 50 nm to less than about 200 nm.

A "headgroup-derivatized" lipid is a lipid which, when present in a liposomal lipid bilayer with an ether lipid, can buffer the toxicity of the ether lipid, that is, can decrease the ether lipid's toxicity, such that it is generally less toxic than the free form of the ether lipid. Headgroup-derivatized lipids generally are amphipathic lipids comprising one or more hydrophobic acyl chains, and a polar headgroup to which a chemical moiety has been attached. The acyl chains typically contain from 4 to 24 carbon atoms, and can be saturated or unsaturated. Preferred acyl chains are those which can adopt compatible packing configurations with the hydrophobic portions of other lipids present in the bilayer, and which can interact with an ether lipid such that release of the ether lipid from the bilayer is inhibited and ether lipid toxicity is buffered. Preferred polar groups are those, such as ethanolamine, to which chemical moieties can be attached. Suitable chemical moieties are those, such as dicarboxylic acids, gangliosides, polyethylene glycols, polyakyl ethers and the like, preferably dicarboxylic acids, which can be attached to polar groups and which give rise to headgroup-derivatized lipids. Means of identifying chemical moieties suitable for attachment to groups such as ethanolamine, for example by subjecting derivatized lipids to in vitro and in vivo toxicity testing, are well known to, and readily practiced by, ordinarily skilled artisans given the teachings of this invention. Means of attaching chemical moieties to polar groups are also well known to, and readily practiced by, ordinarily skilled artisans.

Preferred acyl chains, which include palmitate and oleate chains, have from 16 to 20 carbon atoms. More preferably, one or more of the acyl chains, such as an oleate chain, is unsaturated. The preferred polar group is ethanolamine. Moities preferred for attachment of ethanolamine are dicarboxylic acids, including those which are saturated, or unsaturated, and which have from 4 to 24 carbon atoms, such as glutaric, sebacic, succinic and tartaric acid. More preferably, the dicarboxylic acid is glutaric acid. Accordingly, preferred headgroup-derivatized lipids are phosphatidylethanolamine-dicarboxylic acids such as dipalmitoyl phosphatidylethanolamine-glutaric acid (DPPE-GA), palmitoyloleoyl phosphatidylethanolamine-glutaric acid ((POPE-GA) and dioleoyl phosphatidylethanolamine-glutaric acid (DOPE-GA). Most preferably, the derivatized lipid is DOPE-GA. Typically, the headgroup-derivatized lipid comprises from about 5 mole percent to about 50 mole percent of the liposome's lipid bilayer.

Toxicity buffering capacities of headgroup-derivatized lipids can be determined by a number of in vitro and in vivo testing methods well known to, and readily practiced by, ordinarily skilled artisans, given the teachings of this invention. For example, ether lipid-induced red blood cell (RBC) hemolysis can be examined in vitro by combining an ether lipid with an RBC suspension, incubating the combination, and then quantitating the percentage of RBC lysis by spectrophotometry. The data presented below show that the concentration of free ether lipid inducing hemolysis of five percent of an RBC suspension (Hl₅) was 5.2 micromolar, while the Hl₅ values for ether lipids in liposomes also having a headgroup-derivatized lipid ranged from 8.9 micromolar to 106 micromolar.

Additionally, LD₅₀'s for ether lipids can be determined in vivo by injecting a range of ether lipid doses into sample groups of suitable test animals, e.g., mice; the number of animals expiring in each group is determined, and the ether lipid dose at which fifty percent of the animals in expire is determined. The data presented below show that at liposomal ether lipid doses of 100 and 130 mg of ether lipid/kg body weight, none of the treated animals expired, while at a 100 mg/kg dose of free ether lipid, five of five mice in the tested group expired. The LD₅₀ for free ether lipid (ET-18-OCH₃) was about 32.2 mg of the ether lipid per kg of body weight, while the LD₅₀ for liposomal ether lipid was greater than 130 mg per kg.

Therapeutic window "TW" equals Hl₅/Gl₅₀ ("Gl₅₀" equals the dose of an agent inducing fifty percent growth inhibition in a population of cells exposed to the agent). Generally, when a bioactive agent's TW is less than 1, the agent cannot be administered to achieve 50% growth inhibition without causing generally unacceptable levels of hemolysis. Generally, when the TW is about 1 or greater, the bioactive agent can be administered without inducing generally unacceptable levels of hemolysis. Typically, the higher the TW value, the more therapeutically effective is the agent tested. Ether lipid liposomes having bilayers comprising headgroup-derivatized lipids can have TW's of greater than 1. Preferably, the TW of an ether lipid in a liposomal bilayer also comprising a headgroup-derivatized lipid is greater than about 1.5, more preferably, greater than about 2 and still more preferably, greater than about 3.

The following table presents TW's for free and liposomal ether lipids (1-O-octadecyl-2-O-methyl-sn-glycero-3-phosphocholine) in connection with A549 and MCF7 human cancer cells.

| | TW | |
|---|---|---|
| Formulation | A549 | MCF7 |
| Free EL | 0.78 | 0.26 |
| DOPC:Chol:DOPE-GA:EL | 7.36 | 3.61 |
| EPC:Chol:POPE-GA:EL | 2.77 | 0.96 |
| Chol:DPPG:EL | 2.14 | >1.04 |

Data presented in the Examples below show that the hemolytic activity of EL decreased upon liposomal entrapment with a headgroup-derivatized lipid. The ether lipid concentrations inducing 50% red blood cell hemolysis (Hl₅₀) were: free EL, 13 µM: EL-Lip-1: 43 µM; EL-Lip-2, >180 µM. Ether lipid concentrations inducing 50% growth inhibition of A549 cancer cells (Gl₅₀) were similar: free EL, 10.2 µM; EL-Lip-1, 12.4 µM; EL-Lip-2, 14.1 µM. Furthermore, the anti-metastatic activity of free EL and EL-Lip-1 against Lewis lung cancer in mice was compared. EL-Lip-1 was found to inhibit spontaneous lung metastasis, whereas free EL had only limited effectiveness (Control: 28 ± 4.9 lung nodules; free EL: 27.5 ± 5; EL-Lip-1: 8.1 ± 6.5).

Headgroup-derivatized lipids can also be circulation-enhancing lipids, that is, the modifications directed to lipid toxicity buffering can also be directed to circulation enhancement; derivatized lipids can inhibit clearance of liposomes from the circulatory systems of animals to which they have been administered. Liposomes are generally believed to be cleared from an animal's body by way of its reticuloendothelial system (RES). Avoiding RES clearance means that the frequency of liposome administration can be reduced, and that less of a liposome-associated bioactive agent need be administered to achieve desired serum levels of the agent. Enhanced circulation times can also allow targeting of liposomes to non-RES containing tissues. Liposome outer surfaces are believed to become coated with serum proteins, such as opsonins, in animals' circulatory systems.

Without intending in any way to be limited by theory, it is believed that liposome clearance can be inhibited by modifying the outer surface of liposomes such that binding of serum proteins thereto is generally inhibited. Effective surface modification, that is, alterations to the outer surfaces of liposomes which result in inhibition of opsonization and RES uptake, is believed to be accomplished by incorporating into liposomal bilayers lipids whose polar headgroups have been derivatized by attachment thereto of a chemical moiety which can inhibit the binding of serum proteins to liposomes such that the pharmacokinetic behavior of the liposomes in the circulatory systems of animals is altered (see, e.g., Blume et al. (1993); Gabizon et al. (1993); Park et al. (1992); Woodle et al. U.S. Patent No. 5,013,556; U.S. Patent No. 4,837,028; and U.S. Patent Appln. No. 08/065,928, filed May 21, 1993).

The ether lipid has the chemical formula: 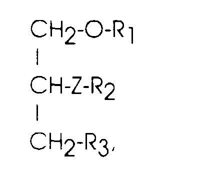 wherein R₁ is Y₁Y₂, Y₂ is CH₃ or CO₂H and Y₁ is (CH₂)ₙ₁(CH=CH)ₙ₂(CH₂)ₙ₃(CH=CH)ₙ₄(CH₂)ₙ₅(CH=CH)ₙ₆(CH₂)ₙ₇ (CH=CH)ₙ₈(CH₂)ₙ₉. n1 is equal to zero or an integer of from 3 to 23; n3 is equal to zero or an integer of from 1 to 21; n5 is equal to zero or an integer of from 1 to 18; n7 is equal to zero or an integer of from 1 to 15; n9 is equal to zero or an integer of from 1 to 12; and each of n2, n4, n6 and n8 is independently equal to zero or 1. The sum of n1 +2n2 +n3 +2n4 +n5 +2n6 + n7 +2n8 +n9 is equal to an integer of from 3 to 23. Accordingly, a hydrocarbon is attached, through an O atom at the 1 position, to the glycerol backbone of the ether lipid. The hydrocarbon can have a a terminal CH₃ or CO₂H group; preferably, the terminal group is a methyl group. The hydrocarbon is preferably saturated, that is, it preferably has no double bonds between adjacent carbon atoms, each of n2, n4, n6 and n8 are equal to zero, and R₁ is (CH₂)ₙ₁CH₃. More preferably, R₁ is (CH₂)₁₇CH₃.

Alternatively, the hydrocarbon can have one or more double bonds, that is, it can be unsaturated. and one or more of n2, n4, n6 and n8 can be equal to 1. When the unsaturated hydrocarbon has one double bond, n2 is equal to 1, n4, n6 and n8 are each equal to zero and Y₁ is (CH₂)ₙ₁CH=CH(CH₂)ₙ₃. n1 is zero or an integer of from 1 to 21, and n3 is also zero or an integer of from 1 to 21, at least one of n1 or n3 not being equal to zero. When the hydrocarbon has two double bonds, n2 and n4 are each equal to 1, n6 and n8 are each equal to zero, and Y₁ is is (CH₂)ₙ₁CH=CH(CH₂)ₙ₃CH=CH(CH₂)ₙ₅. n1 and n5 are each independently zero or an integer of from 1 to 18, and n3 is an integer of from 1 to 16. When the hydrocarbon has three double bonds, n2, n4 and n6 are each equal to 1, n8 is equal to zero, and Y₁ is (CH₂)ₙ₁CH=CH(CH₂)ₙ₃CH=CH(CH₂)ₙ₅CH=CH (CH₂)ₙ₇. n1 and n7 are each independently zero or an integer of from 1 to 15, and n3 and n5 are each equal to an integer of from 1 to 15. When the unsaturated hydrocarbon has four double bonds, each of n2, n4, n6 and n8 is equal to 1, and Y₁ is (CH₂)ₙ₁CH=CH(CH₂)ₙ₃CH=CH(CH₂)ₙ₅CH=CH(CH₂)ₙ₇ CH=CH(CH₂)ₙ₉. n1 and n9 are each independently zero or an integer of from 1 to 12; n3, n5 and n7 are each independently an integer of from 1 to 12.

Z is preferably O, but can also be S. R₂ can also be Y₁Y₂, and can be the same or different than R₁. Preferably, R₂ is (C(X₁)ₙ₁₀(X₂)ₙ₁₁)ₙ₁₂CX₃X₄X₅. Each of X₁, X₂, X₃, X₄, and X₅ is independently H or F, but is preferably H, n10 is equal to zero, 1 or 2, n11 is equal to zero, 1, or 2; and n12 is equal to zero or an integer of from 1 to 23. Preferably, n12 is equal to zero, in which case, R₂ is CX₃X₄X₅. X₃, X₄ and X₅ are preferably H, and R₂ is preferably CH₃. When n12 is not zero, the sum of n10 + n11 is 2, n12 is preferably equal to 1, and R₂ can be CH₂CH₃, CH₂CF₃ or CF₂CF₃.

R₃ is R₅-P(O)₂-O-R₆, R₅ being O, S or NH, but preferably O. R₆ can be CH₂CH₂N(CH₃)₃ (phosphorylcholine) CH₂CH₂NH₂ (phosphorylserine), CH₂CH(OH)CH₂OH (phosphorylglycerol), or CH₂CH₂NHC(O)R₇, but is preferably phosphorylcholine. R₃ is therefore preferably, but not necessarily, -O-P(O)₂-O-CH₂CH₂N(CH₃)₃. R₇ is Y₂CH₃ or Y₂CO₂H.

Accordingly, the preferred ether lipid is: 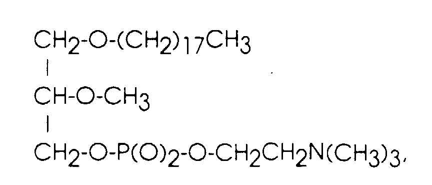 that is, 1-O-octadecyl-2-O-methyl-sn-glycero-3-phosphocholine ("ET-18-OCH₃").

The liposome can comprise additional lipids, that is, one or more lipids in addition to the headgroup-derivatized lipid and ether lipid. Additional lipids are selected for their ability to adapt compatible acyl chain packing conformations with the other components of the lipid bilayer such that the lipid constituents are tightly packed and release of the lipids from the bilayer is inhibited. Lipid-based factors contributing to compatible packing conformations are well known to ordinarily skilled artisans. Such factors include, without limitation, the acyl chain length and degree of unsaturation, as well as the headgroup size and charge. Accordingly, suitable additional lipids can readily be selected by ordinarily skilled artisans given the teachings of this invention.

Preferred additional lipids include neutral lipids such as the phosphatidylcholines (PC) egg phosphatidylcholine (EPC), palmitoyloleoyl phosphatidylcholine (POPC) and dioleoyl phosphatidylcholine (DOPC). Preferred PC's have one or two acyl chains, such as DOPC which has two unsaturated chains. The bilayer of this invention's liposome can also comprise a sterol. Sterols generally affect the fluidity of lipid bilayers (see, for example, Lewis and McElhaney (1992) and Darnell et al. (1986)) Accordingly, sterol interactions with surrounding hydrocarbon chains generally inhibit emigration of these chains from the bilayer. The sterol component of this invention's bilayer is preferably, but not necessarily, cholesterol. The sterol can also be cholesterol sulfate or cholesterol hemisuccinate. The liposome can also comprise a circulation-enhancing lipid, that is, such a lipid in addition to the headgroup-derivatized lipid, which can, but is not necessarily, a circulation-enhancing lipid.

Preferably, the liposome of this invention has a bilayer comprising an ether lipid, a headgroup-derivatized lipid, a sterol and a neutral lipid. Preferably, the ether lipid is ET-18-OCH₃, the headgroup-derivatized lipid is a PE-dicarboxylic acid, the sterol is cholesterol and the neutral lipid is a PC. Most preferably, the PE-dicarboxylic acid is DOPE-GA, and the PC is DOPC. Typically, the bilayer comprises from about 10 to about 30 mole percent ether lipid, from about 5 to about 20 mole percent of the headgroup-derivatized lipid, from about 10 to about 50 mole percent of the sterol and from about 10 to about 50 mole percent of the neutral lipid. In a particularly preferred embodiment of this invention, the bilayer comprises about 20 mole percent ET-18-OCH₃, about 10 mole percent DOPE-GA, about 30 mole percent cholesterol and about 40 mole percent DOPC.

The liposome can comprise an additional bioactive agent, that is, a bioactive agent in addition to the ether lipid. A "bioactive agent" is any compound or composition of matter that can be administered to animals, preferably humans. Such agents can have biological activity in animals; the agents can also be used diagnostically in the animals. Bioactive agents include therapeutic and imaging agents. Bioactive agents which may be associated with liposomes include, but are not limited to: antiviral agents such as acyclovir, zidovudine and the interferons; antibacterial agents such as aminoglycosides, cephalosporins and tetracyclines; antifungal agents such as polyene antibiotics, imidazoles and triazoles; antimetabolic agents such as folic acid, and purine and pyrimidine analogs; antineoplastic agents such as the anthracycline antibiotics and plant alkaloids; sterols such as cholesterol; carbohydrates, e.g., sugars and starches; amino acids, peptides, proteins such as cell receptor proteins, immunoglobulins, enzymes, hormones, neurotransmitters and glycoproteins; dyes; radiolabels such as radioisotopes and radioisotope-labelled compounds; radiopaque compounds; fluorescent compounds; mydriatic compounds; bronchodilators; local anesthetics; and the like. Liposomal bioactive agent formulations can enhance the therapeutic index of the bioactive agent, for example by buffering the agent's toxicity. Liposomes can also reduce the rate at which a bioactive agent is cleared from the circulation of animals. Accordingly, liposomal formulation of bioactive agents can mean that less of the agent need be administered to achieve the desired effect. Additional bioactive agents preferred for the liposome of this invention include antimicrobial, anti-inflammatory and antineoplastic agents, or therapeutic lipids, for example, ceramides. Most preferably, the additional bioactive agent is an antineoplastic agent.

Liposomes can be loaded with one or more biologically active agents by solubilizing the agent in the lipid or aqueous phase used to prepare the liposomes. Alternatively, ionizable bioactive agents can be loaded into liposomes by first forming the liposomes, establishing an electrochemical potential, e.g., by way of a pH gradient, across the outermost liposomal bilayer, and then adding the ionizable agent to the aqueous medium external to the liposome (see Bally et al. U.S. Patent No. 5,077,056 and WO86/01102).

The liposome of this invention can be dehydrated, stored and then reconstituted such that a substantial portion of their internal contents are retained in the liposomes. Liposomal dehydration generally requires use of a hydrophilic drying protectant (see U.S. Patent Nos. 4,229,360 and 4,880,635). This hydrophilic compound is generally believed to prevent the rearrangement of the lipids in the liposome, so that the size and contents are maintained during the drying procedure and through rehydration. such that the liposomes can be reconstituted. Appropriate qualities for such drying protectants are that they be strong hydrogen bond acceptors, and possess stereochemical features that preserve the intramolecular spacing of the liposome bilayer components. Saccharide sugars, preferentially mono- and disaccharides, are suitable drying protectants for liposomes. Alternatively, the drying protectant can be omitted if the liposome preparation is not frozen prior to dehydration, and sufficient water remains in the preparation subsequent to dehydration.

Also provided herein is a pharmaceutical composition comprising a pharmaceutically acceptable carrier and the liposome of this invention. "Pharmaceutically acceptable carriers" as used herein are generally intended for use in connection with the administration of lipids and liposomes, including liposomal bioactive agent formulations, to animals, including humans. Pharmaceutically acceptable carriers are generally formulated according to a number of factors well within the purview of the ordinarily skilled artisan to determine and account for, including without limitation: the particular liposomal bioactive agent used, its concentration, stability and intended bioavailability; the disease, disorder or condition being treated with the liposomal composition; the subject, its age, size and general condition; and the composition's intended route of administration, e.g., nasal, oral, ophthalmic, topical, transdermal, vaginal, subcutaneous, intramammary, intraperitoneal, intravenous, or intramuscular (see, for example, Nairn (1985)). Typical pharmaceutically acceptable carriers used in parenteral bioactive agent administration include, for example, D5W, an aqueous solution containing 5% weight by volume of dextrose, and physiological saline. Pharmaceutically acceptable carriers can contain additional ingredients, for example those which enhance the stability of the active ingredients included, such as preservatives and antioxidants.

Further provided is a method of administering an ether lipid to an animal which comprises administering to the animal a pharmaceutical composition comprising a pharmaceutically acceptable carrier and a liposome having a lipid bilayer which comprises a headgroup-derivatized lipid and the ether lipid. The ether lipid has the chemical formula:

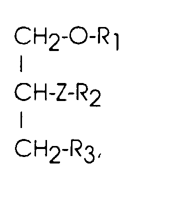

Preferably the animal is a human, and the ether lipid is administered intravenously. Preferably, the liposome has a diameter of less than about 200 nm, more preferably, from greater than about 50 nm to less than about 200 nm.

The headgroup-derivatized lipid is preferably a phosphatidylethanolamine-dicarboxylic acid, the acid preferably being glutaric acid and the PE preferably being DPPE, POPE or DOPE. More preferably, the headgroup-derivatized lipid is DOPE-GA. The headgroup-modified lipid can also be a circulation-enhancing lipid.

Preferably, a saturated hydrocarbon, more preferably CH₃(CH₂)₁₇-is attached at the 1 position of the ether lipid through an O; preferably, a methyl group is attached at the 2 position through an O; and preferably, the polar group at the 3 position is phosphorylcholine. Accordingly, the preferred ether lipid is 1-O-octadecyl-2-O-methyl-3-sn-glycero-phosphocholine ("ET-18-OCH₃"). Preferably, the liposome's lipid bilayer also comprises a sterol, more preferably, cholesterol, and a neutral lipid, such as a PC, and preferably DOPC. The bilayer can also comprise a circulation-enhancing lipid.

Preferably, the liposome used in the method of this invention has a lipid bilayer comprising ET-18-OCH₃, DOPE-GA, cholesterol and DOPC. More preferably, the bilayer comprises about 40 mole percent DOPC. about 30 mole percent cholesterol, about 10 mole percent of DOPE-GA and about 20 mole percent of the ether lipid.

Ether lipids are generally believed to be selectively cytotoxic to tumor cells. Accordingly, the method of this invention can be used to treat animals afflicted with a cancer, including, but not limited to, a carcinoma, leukemia, myeloma, neuroblastoma, or a sarcoma, of the lung, brain, ovarian, colon or breast. Generally, liposomal ether lipids can be used to treat cancers treated with free, that is, nonliposomal, ether lipids. However, encapsulation of an ether lipid in a liposome can enhance its therapeutic index, and therefore make the liposomal ether lipid a more effective treatment. The method is practiced by administering to the animal an antineoplastic effective amount of the liposomal ether lipid of this invention. Administration of the liposomal ether lipid can inhibit metastases of cancerous cells, or their invasion of tissues.

For the purposes of this invention, "anticancer effective amounts" of liposomal ether lipids are amounts effective to inhibit, ameliorate, lessen or prevent establishment, growth, metastasis or invasion of one or more cancers in animals to which the ether lipids have been administered. Anticancer effective amounts are generally chosen in accordance with a number of factors, e.g., the age, size and general condition of the subject, the cancer being treated and the intended route of administration, and determined by a variety of means, for example, dose ranging trials, well known to, and readily practiced by, ordinarily skilled artisans given the teachings of this invention. Antineoplastic effective amounts of the liposomal ether lipid of this invention are generally expected to be about the same as such amounts of free, nonliposomal, ether lipids, and range from about 0.1 mg of the ether lipid per kg of the body weight of the animal treated to about 1000 mg of the ether lipid per kg of body weight. Preferably, the antineoplastic effective amount of the ether lipid is from about 1 mg of the ether lipid per kg of body weight to about 500 mg per kg. More preferably, the anticancer effective amount of the liposomal ether lipid is from about 5 mg/kg to about 100 mg/kg.

The method of this invention can also be used to administer an ether lipid to an animal afflicted with an anti-inflammatory disorder. Inflammatory disorders treatable by the method of this invention include, without limitation: arthritic conditions such as gout, rheumatoid arthritis, filary arthritis and Lyme disease; asthmatic disorders; allergic reactions; and the like. Animals are treated for such disorders by administering an anti-inflammation effective amount of the ether lipid to the animal.

Inflammation is a process of cytological and histological reactions occurring in affected blood vessels, and surrounding tissues, in response to an injury (see, e.g., Stedman's Medical Dictionary (Illustrated) (1982)). Inflammatory responses to such stimuli include local reactions and resulting morphological changes, destruction or removal of injurious materials and activation of repair mechanisms. Thus, inflammation can be part of the process by which animals heal themselves. However, inflammation can also occur in response to abnormal physiological stimuli, and can cause problems in the body. Joints, for example, become inflamed in arthritic conditions such as gout, filary arthritis, rheumatoid arthritis and Lyme disease (see, e.g., Stedmans Medical Dictionary (Illustrated), *supra* at pages 123-124). These states may be characterized by the extravasation of cells, i.e, the egress of cells from the circulation into the inflamed area. Agents which can inhibit such extravasation, or which can otherwise inhibit inflammatory responses to abnormal physiological stimuli, can be used to ameliorate the inflammation.

An "anti-inflammation effective amount" of a liposomal ether lipid is any amount of the ether lipid effective to ameliorate, inhibit or prevent inflammatory responses or reactions in animals afflicted with conditions characterized by abnormal inflammation, i.e., inflammation which is in response to abnormal physiological stimuli and which is not part of the body's normal repair processes in response to an injury. Anti-inflammation effective amounts of ether lipids are generally from about 0.1 mg of the ether lipid per kg of the body weight of the animal treated to about 1000 mg of the ether lipid per kg of body weight.

The method of this invention can also comprise administration of an additional biologically active agent to an animal, that is, a biologically active agent in addition to the ether lipid administered. This additional biologically active agent, e.g., an antimicrobial agent, anti-inflammatory agent, antineoplastic agent, therapeutic lipid, or hematopoietic cell growth stimulating agent can be associated, or unassociated, with the liposome. "Association" of a biologically active agent with a liposome means entrapment of the agent in an aqueous compartment or lipid bilayer of the liposome, or association, for example, by means of complex formation, of the agent with the surface of the inner or outer monolayer of the bilayer Preferably, the additional biologically active agent associated with the liposome is an antineoplastic agent, such as an anthracycline antibiotic, antimetabolite or vinca alkaloid. Unassociated bioactive agents can be administered concurrently with, or at different times than, liposome administration. Preferred unassociated biologically active agents are red blood cell growth stimulating agents, e.g., erythropoietin, or interleukin 3 (IL-3) and granulocyte/macrophage colony stimulating factor (GM-CSF).

This invention will be better understood from the following examples. However, those of ordinary skill in the art will readily understand that these examples are merely illustrative of the invention as defined in the claims which follow thereafter.

### EXAMPLES

### Example 1

### Preparation

**Chemicals:** Ether lipid (EL), egg phosphatidylcholine (EPC), distearoyl phosphatidylcholine (DSPC), dipalmitoyl phosphatidylethanolamine-glutaric acid (DPPE-GA), palmitoyloleoyl phosphatidylethanolamine-glutaric acid (POPE-GA), and dioleoyl phosphatidylethanolamine-glutaric acid (DOPE-GA) were obtained from Avanti Polar Lipids (Birmingham, Alabama). Cholesterol (Chol) was purchased from Sigma Chemical Company U.S.A. Dulbecco's phosphate buffered saline (D-PBS) was purchased from Gibco BRL Life Technologies Inc.(Grand Island, New York, U.S.A). All other reagents were of the highest purity available.

**Liposomes:** 'Empty' liposomes were prepared by the solvent evaporation method. Liposomes containing EL were prepared by first dissolving EL in chloroform and mixing in EPC/Chol, DOPC/Chol or DSPC/Chol at various mole ratios, with and without 10 mole % of either DPPE-GA, POPE-GA or DOPE-GA in chloroform and methanol (2:1 v/v). The organic solvent was removed under vacuum using a rotary evaporator and the thin dried film was hydrated with a buffer solution of DPBS. The resulting preparations were extruded 10 times through 0.1µm double stacked Nucleopore filters using an extruder device (Lipex Biomembrane Vancouver, BC, Canada). Sizes of liposomes were determined by light scattering a Nicomp Model 370 Submicron Particle Sizer. For all studies, liposomes had a mean diameter between 96-126 nm.

### Example 2

### Red Blood Cell (RBC) Hemolysis Assay

A 4% suspension of red blood cells (RBCs), 0.5 ml washed three times in PBS, was incubated with various concentrations (serially diluted) of "empty" liposomes, that is, liposomes without ether lipids or other biologically active agents, free (non-liposomal) ether lipid or liposomal ether lipid, prepared as described above. These samples were vortexed on a 37 deg. C. agitator for 20 hours, and were then centrifuged for 10 minutes at 3000 rpm. 0.2 ml of the resulting supernatant was diluted to 1 ml with water, and the percentage hemolysis in the sample was quantitated by spectrophotometric examination at 550 nm. Release of hemoglobin by hypotonic lysis of an equal number of RBC by water was taken as 100% positive control; PBS served as negative control. The ether lipid used in the formulations was 1-O-octadecyl-2-O-methyl-sn-glycero-3-phosphocholine (ET-18-O-CH₃).

Results from these studies are presented in Table 1 (see below), the first column indicating the components of the formulation tested for hemolytic activity, the middle column indicating the molar ratios of the respective components, and the third column indicating the lipid concentration (micromolar) at which 5% hemolysis was observed (Hl₅).

**TABLE 1**

| Hemolytic Activity | | |
|---|---|---|
| Composition | Molar Ratio | Hl₅ |
| **Free EL** | N/A | 5.2 |
| | | |
| **Liposomal Ether Lipid:** | | |
| | | |
| EPC/Chol/EL | 4:3:3 | 20.6 |
| | | |
| EPC/Chol/DPPE-GA/EL: | 1:3:1:5 | 8.9 |
| | 3:3:1:3 | 14.1 |
| | 4:3:1:2 | 19.0 |
| | 5:3:1:1 | 32.8 |
| | | |
| EPC/Chol/POPE-GA/EL: | 1:3:1:5 | 13.9 |
| | 3:3:1:3 | 20.1 |
| | 4:3:1:2 | 29.9 |
| | 5:3:1:1 | 31.1 |
| | | |
| EPC/Chol/DOPE-GA/EL | 4:3:1:2 | 58.7 |
| | | |
| DOPC/Chol/DOPE-GA/EL | 4:3:1:2 | 106.0 |
| | | |
| Chol/DPPG/EL | 5:3:2 | 71.8 |
| | | |
| DSPC/Chol/EL | 4:3:3 | 20.4 |
| | | |
| DSPC/Chol/DPPE-GA/EL: | 1:3:1:5 | 9.0 |
| | 3:3:1:3 | 10.5 |
| | 4:3:1:2 | 17.9 |
| | 5:3:1:1 | 19.8 |

### Example 3

### In Vitro Murine Cancer Cell Growth Inhibition Studies

The specific inhibition of *in vitro* proliferation of various cancer cells by free, liposomal-ET-18-OCH3 or empty (EL free) liposomes was monitored by a Sulforhodamine B (SRB) assay. In brief, 5000 Lewis lung cancer (LLC), P388 or P388-adriamycin resistant (ADR) cells/well were separately plated onto 96 well flat-bottomed microtiter plates in a RPMI-1640 medium supplemented with 10% FBS, and kept at 37°C in a humidified atmosphere of 5% CO₂. After 24 hours, cells were exposed to various concentrations of either empty (EL free) liposomes, DPBS, free ether lipid or liposomal-ether lipid and cultured for another 48 hours at 37°C. Cells treated with various formulations of ET-18-OCH3 were fixed by adding 50 µl of cold 50% (wt/vol) trichloroacetic acid (TCA) and incubated at 4°C for 1 hour. Plates were rinsed five times with deionized water and left to air dry over night. 100 µl of SRB (0.4% wt/vol in 1% acetic acid ) was added and incubated at room temperature for 10-15 minutes. Unbound SRB was removed by washing five times with 1% of acetic acid. Plates were air dried and bound SRB was solubilized with Tris buffer, and optical densities were read at 551 nm and 50% growth inhibition (Gl₅₀) was calculated by formula 50 x ((T - T0)/(C-T0)) = 50, where control optical density is (C), test optical density (T) and optical density at time zero is (T0).

Results are presented in Table 2 (see below) as the concentration of ether lipid which achieved fifty percent growth inhibition (Gl₅₀), with the first column indicating the composition of the formulation tested, the second column indicating the molar ratios of the respective formulation components and the third, fourth and fifth columns being the Gl₅₀ data for the LLC, P388 and P388-ADR cultures, respectively. The ether lipid used in the formulations was 1-O-octadecyl-2-O-methyi-sn-giycero-3-pnospnocholine.

**TABLE 2**

| Growth Inhibition (Gl₅₀) of Murine Cancer Cells by Ether Lipid Formulations | | | | |
|---|---|---|---|---|
| Composition | Molar Ratio | LLC | P388 | P388-ADR |
| **Free EL** | N/A | 26.5±5.1 | 4.2±0.2 | 6.1±1.6 |
| **Liposomal EL:** | | | | |
| EPC/Chol/EL | 4:3:3 | 40.6 | ----- | ----- |
| EPC/Chol/POPE-GA/EL: | | | | |
| | 1:3:1:5 | 44.5±3 | 5.6±0.4 | 10.8±0.4 |
| | 3:3:1:3 | 63.2±4 | 5.2±0.1 | 9.8±0.4 |
| | 4:3:1:2 | 73.6±0.5 | 5.3±0.2 | 9.6+0.4 |
| | 5:3:1:1 | >75 | 5.9±0.5 | 12.3±0.4 |
| EPC/Chol/DPPE-GA/EL: | | | | |
| | 1:3:1:5 | 28.9 | ----- | ------ |
| | 3:3:1:3 | 30.6 | ----- | ------ |
| | 4:3:1:2 | >75 | 5.1±1.3 | 8.2±4.3 |
| | 5:3:1:1 | >60 | ----- | ----- |
| EPC/Chol/DOPE-GA/EL: | | | | |
| | 4:3:1:2 | 71.9 | 7.5 | 13.3 |
| DOPC/Chol/DOPE-GA/EL | | | | |
| | 4:3:1:2 | 67.6±3.3 | 5.7±0.2 | 11.5±0.4 |
| Chol/DPPG/EL | 5:3:2 | >75 | 11.2 | 24.3 |
| DSPC/Chol/EL | 4:3:3 | 36.6 | ----- | ----- |
| DSPC/Chol/DPPE-GA/EL: | | | | |
| | 3:3:1:3 | 25.1 | ----- | ----- |

### Example 4

### In Vitro Human Cancer Cell Growth Inhibition Studies

Human A549 lung cancer, MCF7 and MCF7/ADR cells were plated (5000 cells per well), and after 24 hours incubation, were exposed to various concentrations of ether lipid, either free or liposomal, for 72 hours. Growth inhibition was determined using the SRB assay. Incubation with control liposomes, not containing ether lipid, did not cause growth inhibition at lipid concentrations up to 60 µM. Results are presented in Table 3 (see below) as the concentration of ether lipid which achieved fifty percent growth inhibition (Gl₅₀), with the first column indicating the composition of the formulation tested, the second column indicating the molar ratio of the formulation's constituents, and the third, fourth and fifth columns indicating the results for A549. MCF7 and MCF7/ADR cultures, respectively. The ether lipid used in the formulations was the 1-O-octadecyl-2-O-methyl-sn-glycero-3-phosphocholine.

**TABLE 3**

| Growth Inhibition (Gl₅₀) of Human Cancer Cells by Ether Lipid Formulations | | | | |
|---|---|---|---|---|
| Composition | Molar Ratio | A549 | MCF7 | MCF7/ADR |
| **Free EL** | N/A | 6.7±2.5 | 20.2±7.7 | 28.4±4.5 |
| **Liposomal EL** | | | | |
| EPC:Chol:POPE-GA:EL | 1:3:1:5 | 9.9±0.1 | 19.1±1.8 | 34.9±1.8 |
| | 3:3:1:3 | 11.8±0.2 | 29.1+1.7 | 41±0.4 |
| | 4:3:1:2 | 10.8±0.4 | 31.2±2.2 | 45.1±0.7 |
| | 5:3:1:1 | 18.3±0.1 | >66 | >75 |
| EPC:Chol:DPPE-GA:EL: | | | | |
| | 4:3:1:2 | 9.2±3.4 | 31.6±0.5 | 55.7±0.1 |
| EPC:Chol:DOPE-GA:EL: | | | | |
| | 4:3:1:2 | 15.8 | 36 | >68.6 |
| DOPC:Chol:DOPE-GA:EL: | | | | |
| | 4:3:1:2 | 14.4±0.4 | 29.4±14.6 | >63.4 |
| Chol:DPPG:EL | 5:3:2 | 33.5 | >75 | >75 |

### Example 5

### In Vivo Toxicity Studies

Groups of female C57/BL6 mice (5 /group, weight 20-22 gm) were injected with various doses of free (12.5-200 mg/kg) and liposomal-ether lipid (12.5-200 mg/kg) in PBS via the tail vein. Ethanol was also injected in a separate group of mice. The mice were observed for immediate, acute toxicity; results are presented in Table 4 (see below).

**TABLE 4**

| *IN VIVO* TOXICITY | | |
|---|---|---|
| **Formulation** | **Ether Lipid Dose**(mg/kg) | **# Dead Animals** |
| Ethanol Control | ----- | 0/5 |
| **Free EL:** | | |
| | 200 | 5/5 |
| | 100 | 5/5 |
| | 50 | 4/5 |
| | 25 | 1/5 |
| | 12.5 | 0/5 |
| | 6.25 | 0/5 |
| **Liposomal EL:** | | |
| EPC/Chol/GA/EL (5/3/1/1) | 100 | 0/5 |
| EPC/Chol/GA/EL (4/3/1/2) | 130 | 0/5 |

### Example 6

### In Vivo Efficacy Studies

Lewis Lung cancer cells(LLC) were obtained from the ATCC and maintained in RPMI medium. Four groups (10/group) of female C57/BL6 mice (body weight 18-22 gm) were injected with 2x10⁵ cells of LLC suspended in 0.2 ml sterile PBS via the tail vein on day 0. On days 1,3,5,7,9 following tumor injection, the first and second groups received either 25 mg/kg free EL or liposomal-EL via the tail vein. The third and fourth groups were administered either the same amount of lipid dosed as EL free liposomes with 0.2 ml PBS. The in *vivo* anti-metastatic activity of free and liposomal-EL was evaluated on the basis of number of tumor nodules present in the lungs of experimental groups of mice. Results are presented in Figures 1 and 2.

### References Cited

### U.S. Patent Documents

4,159,988, 4,163,748, 4,235,871, 4,382,035, 4,522,803, 4,588,578, 4,734,225, 4,804,789, 4,837,028, 4,920,016, 4,975,282, 5,008,050, 5,013,556, 5,030,453, 5,059,421, 5,077,056, 5,169,637, 3,752,886

### Foreign Patent Documents

W089/008846, 1,583,661,4,132,345

### Others

Bazill and Dexter, Cancer Res. 50: 7505 (1990).
Berdel, Br. J. Cancer 64:208 (1991).
Berdel, Onkologie 13: 245 (1990).
Bhatia and Hadju, Lipids 26(12): 1424 (1991).
Blume et al., Biochim. Biophys. Acta. U1149U: 180 (1993)
Darnell et al, Molecular Cell Biology, Scientific American Books, Inc. (1986), New York, pp. 573-575.
Deamer and Uster, "Liposome Preparation: Methods and Mechanisms," in: Liposomes (M. Ostro, ed.), Marcel Dekker, Inc., New York (1983), pp. 27-52.
Dietzfelbinger et al, Cancer Res. 53: 3747 (1993).
Gabizon et al., Pharm. Res. 10(5): 703 (1993)
Layton et al., Eur. J. Cancer 16: 64 (1980).
Lewis and McElhaney, "The Mesomorphic Phase Behavior of Lipid Bilayers," in The Structure of Biological Membranes (P. Yeagle, ed.), CRC Press, Inc. (1992), Boca Raton, Fl., pp. 73-155, at pp. 123-126.
Muschiol et al., Lipids 22(11): 930 (1987).
Nairn, in: Remington's Pharmaceutical Science (A. Gennaro, ed.), Mack Publishing Co., Easton, PA, (1985), pp. 1492-1517.
Paphadjopoulos et al., Biochem. Biophys. Acta 135: 624 (1968). Park et al., Biochim. Biophys. Acta. 1108: 257 (1992).
Powis et al., Cancer Res. 52: 2835 (1992).
Powis et al., Cancer Res. 50: 2203 (1990).
Reed et al., Life Sci. 49: 1221 (1991).
Runge et al., JNCI. 64(6): 1301 (1980).
Stedman's Medical Dictionary (Illustrated) (24th edition, J. V. Basmajian et al., eds.), Williams and Wilkins, Baltimore, MD (1982), pp. 707-708. Tritton and Hickman, Cancer Cells 2(4): 95 (1990).
Workman, Cancer Cells 3(8): 315 (1991).
Workman et al., Biochem. Pharmacol. 41(2): 319 (1991).
Zeisig et al., Anti-Cancer Drugs 4: 57 (1993).

## Claims

1. A liposome having a lipid bilayer comprising an ether lipid and a headgroup-derivatized lipid, wherein the ether lipid has the chemical formula: 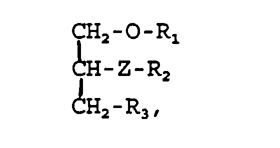
wherein R₁ is Y₁Y₂, Y₂ is CH₃ or CO₂H and Y₁ is (CH₂)ₙ₁ (CH=CH)ₙ₂(CH₂)ₙ₃(CH=CH)ₙ₄(CH₂)ₙ₅(CH=CH)ₙ₆(CH₂)ₙ₇ (CH=CH)ₙ₈(CH₂)ₙ₉;
wherein the sum of n1 + 2n2 + n3 + 2n4 + n5 + 2n6 + n7 + 2n8 + n9 is equal to an integer of from 3 to 23, n1 is zero or an integer of from 3 to 23, n3 is zero or an integer of from 1 to 21, n5 is zero or an integer of from 1 to 18, n7 is zero or an integer of from 1 to 15, n9 is zero or an integer of from 1 to 12, and each of n2, n4, n6 and n8 is independently zero or 1;
wherein Z is O or S;
wherein R₂ is Y₁Y₂ or (C(X₁)ₙ₁₀(X₂)ₙ₁₁)ₙ₁₂CX₃X₄X₅ ;
wherein each of X₁, X₂, X₃, X₄, and X₅ is independently H or F, n10 is zero, 1 or 2, n11 is zero, 1, or 2, n12 is an integer of from 1 to 23, and the sum of n10 + n11 is equal to 2 ;
wherein R₃ has the formula R₅-P(O)₂-O-R₆, R₅ is 0, S or NH, R₆ is CH₂CH₂N(CH₃)₃, CH₂CH₂NH₂, CH₂CH(OH)CH₂OH, or CH₂CH₂NHC(O)R₇ and wherein R₇ is Y₂CH₃ or Y₂CO₂H.

2. The liposome of claim 1, wherein the liposome has a diameter of less than about 200 nm.

3. The liposome of claim 2, wherein the liposome has a diameter of from greater than about 50 nm to less than about 200 nm.

4. The liposome of claim 1, wherein Y₂ is CH₃.

5. The liposome of claim 4, wherein R₁ is (CH₂)ₙ₁CH₃.

6. The liposome of claim 5, wherein R₁ is (CH₂)₁₇CH₃.

7. The liposome of claim 1, wherein Z is O.

8. The liposome of claim 1, wherein R₂ is (C(X₁)ₙ₁₀(X₂)ₙ₁₁)ₙ₁₂ CX₃X₄X₅.

9. The liposome of claim 8, wherein n12 is one.

10. The liposome of claim 9, wherein n12 is one and R₂ is CH₂CH₃, CH₂CF₃ or CF₂CF₃.

11. The liposome of claim 1, wherein R₃ is O-P(O)₂-O-R₆.

12. The liposome of claim 11, wherein R₃ is -O-P(O)₂-O-CH₂CH₂N (CH₃)₃.

13. A liposome having a lipid bilayer comprising an ether lipid and a headgroup-derivatized lipid, wherein the ether lipid is defined as in claim 1 with the proviso that n12 is 0 or an integer from 1 to 23,
wherein the headgroup-derivatized lipid is a phosphatidylethanolamine-dicarboxylic acid, preferably dioleoyl phosphatidylethanolamine-glutaric acid.

14. The liposome of claim 13, wherein the phosphatidylethanolamine is dipalmitoyl phosphatidylethanolamine, palmitoyloleoyl phosphatidylethanolamine or dioleoyl phosphatidylethanolamine, preferably dioleoyl phosphatidylethanolamine.

15. The liposome of claim 13, wherein the dicarboxylic acid is glutaric, sebacic, succinic or tartaric acid, preferably glutaric acid.

16. A liposome having a lipid bilayer comprising an ether lipid and a headgroup-derivatized lipid, wherein the ether lipid is defined as in claim 1 with the proviso that n12 is 0 or an integer from 1 to 23, wherein the headgroup-derivatized lipid is a circulation-enhancing lipid.

17. The liposome of claim 1, wherein the bilayer comprises a sterol.

18. A liposome having a lipid bilayer comprising an ether lipid and a headgroup-derivatized lipid, wherein the ether lipid is defined as in claim 1 with the proviso that n12 is 0 or an integer from 1 to 23, wherein the bilayer comprises a neutral lipid.

19. The liposome of claim 18, wherein the neutral lipid is a phosphatidylcholine.

20. The liposome of claim 19, wherein the phosphatidylcholine is egg phosphatidylcholine, palmitoyloleoyl phosphatidylcholine or dioleoyl phosphatidylcholine, preferably dioleoyl phosphatidylcholine.

21. A liposome having a lipid bilayer comprising an ether lipid and a headgroup-derivatized lipid, wherein the ether lipid is defined as in claim 1 with the proviso that n12 is 0 or an integer from 1 to 23, comprising a circulation-enhancing lipid.

22. The liposome of claim 21, further comprising a sterol and a neutral lipid.

23. The liposome of claims 16, 18 or 21, wherein the ether lipid is:

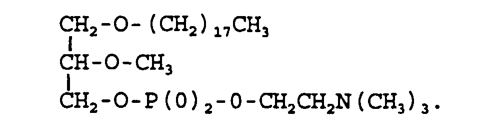

24. The liposome of claim 23, wherein the headgroup-derivatized lipid is dioleoyl phosphatidylethanolamine-glutaric acid.

25. The liposome of claim 17 or 22, wherein the sterol is cholesterol.

26. The liposome of claim 22, wherein the neutral lipid is dioleoyl phosphatidylcholine.

27. The liposome of claim 22, wherein the bilayer comprises about 20 mole percent of the ether lipid, about 10 mole percent dioleoyl phosphatidylethanolamine-glutaric acid, about 30 mole percent cholesterol and about 40 mole percent dioleoyl phosphatidylcholine.

28. The liposome of any one of the claims 1 to 27, comprising an additional bioactive agent.

29. A pharmaceutical composition comprising a pharmaceutically acceptable carrier and the liposome of any one of the claims 1 to 28.

30. A method of preparing a pharmaceutical composition for administering an ether lipid to an animal comprising a pharmaceutically acceptable carrier and a liposome having a lipid bilayer comprising the ether lipid and a headgroup-derivatized lipid, wherein the ether lipid has the chemical formula: 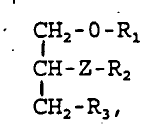
wherein R₁ is Y₁Y₂, Y₂ is CH₃ or CO₂H and Y₁ is (CH₂)ₙ₁(CH=CH)ₙ₂ (CH₂)ₙ₃ (CH=CH)ₙ₄ (CH₂)ₙ₅ (CH=CH)ₙ₆ (CH₂)ₙ₇ (CH=CH)ₙ₈(CH₂)ₙ₉;
wherein the sum of n1 + 2n2 + n3 + 2n4 + n5 + 2n6 + n7 + 2n8 + n9 is equal to an integer of from 3 to 23, n1 is zero or an integer of from 3 to 23, n3 is zero or an integer of from 1 to 21, n5 is zero or an integer of from 1 to 18, n7 is zero or an integer of from 1 to 15, n9 is zero or an integer of from 1 to 12, and each of n2, n4, n6 and n8 is independently zero or 1;
wherein Z is O or S;
wherein R₂ is Y₁Y₂ or (C(X₁)ₙ₁₀ (X₂)ₙ₁₁)ₙ₁₂CX₃X₄X₅ ;
wherein each of X₁, X₂, X₃, X₄, and X₅ is independently H or F, n10 is zero, 1 or 2, n11 is zero, 1, or 2, n12 is an integer of from 1 to 23, and the sum of n10 + n11 is equal to 2;
and wherein R₃ has the formula R₅-P(O)₂-O-R₆, R₅ is O, S or NH, R₆ is CH₂CH₂N(CH₃)₃, CH₂CH₂NH₂, CH₂CH(OH)CH₂OH, or CH₂CH₂NHC(O)R₇ and R₇ is Y₂CH₃ or Y₂CO₂H.

31. The method of claim 30, wherein the animal is a human.

32. The method of claim 30, wherein the animal is afflicted with a cancer and wherein the pharmaceutical preparation comprises an anticancer effective amount of the ether lipid.

33. The method of claim 32, wherein the cancer is a lung, brain, colon, ovarian or breast cancer.

34. The method of claim 32, wherein the anticancer effective amount of the ether lipid is from about 0.1 mg of the ether lipid per kg of the body weight of the animal to which the pharmaceutical composition is administered to about 1000 mg of the ether lipid per kg of body weight.

35. The method of claim 30, wherein the animal is afflicted with an inflammatory disorder and the pharmaceutical preparation comprises an anti-inflammation effective amount of the ether lipid.

36. The method of claim 35, wherein the inflammatory disorder is an arthritic condition, asthmatic disorder or allergic reaction.

37. The method of claim 35, wherein the anti-inflammation effective amount of the ether lipid is from about 0.1 mg of the ether lipid per kg of the body weight of the animal to which the pharmaceutical composition is administered to about 1000 mg of the ether lipid per kg of body weight.

38. The method of claim 30, wherein the pharmaceutical composition comprises an additional biologically active agent.

39. The method of claim 38, wherein the additional biologically active agent is an antineoplastic agent, antimicrobial agent, anti-inflammatory agent, therapeutic lipid or hematopoietic cell growth stimulating agent.

40. The method of claim 30, wherein the liposome has a diameter of from greater than about 50 nm to less than about 200 nm.

41. The method of claim 30, wherein R₁ is (CH₂)₁₇CH₃.

42. The method of claim 30, wherein R₂ is CH₃.

43. The method of claim 30, wherein R₃ is -O-P(O)₂-O-CH₂CH₂N(CH₃)₃.

44. A method of preparing a pharmaceutical composition for administering an ether lipid to an animal comprising a pharmaceutically acceptable carrier and a liposome having a lipid bilayer comprising the ether lipid and a headgroup-derivatized lipid, wherein the ether lipid is defined as in claim 1 with the proviso that n12 is 0 or an integer from 1 to 23, wherein the headgroup-derivatized lipid is phosphatidylethanolamine-dicarboxylic acid, preferably dioleoyl phosphatidylethanolamine-glutaric acid.

45. A method of preparing a pharmaceutical composition for administering an ether lipid to an animal comprising a pharmaceutically acceptable carrier and a liposome having a lipid bilayer comprising the ether lipid and a headgroup-derivatized lipid, wherein the ether lipid is defined as in claim 1 with the proviso that n12 is 0 or an integer from 1 to 23, wherein the bilayer further comprises a sterol and a neutral lipid.

46. The method of claim 45, wherein the headgroup-derivatized lipid is dioleoyl phosphatidylethanolamine-glutaric acid, the sterol is cholesterol, the neutral lipid is dioleoyl phosphatidylcholine and the ether lipid is:

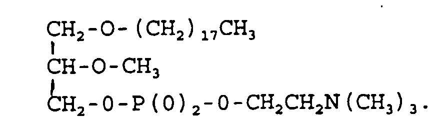

47. The method of claim 46, wherein the bilayer comprises about 20 mole percent of the ether lipid, about 10 mole percent dioleoyl phosphatidylethanolamine-glutaric acid, about 30 mole percent cholesterol and about 40 mole percent dioleoyl phosphatidylcholine.

48. A method of preparing a pharmaceutical composition for administering an ether lipid to an animal comprising a pharmaceutically acceptable carrier and a liposome having a lipid bilayer comprising the ether lipid and a headgroup-derivatized lipid, wherein the ether lipid is defined as in claim 1 with the proviso that n12 is O or an integer from 1 to 23, wherein the liposome comprises a circulation-enhancing lipid.

## Patentansprüche

1. Liposom mit einer Lipid-Doppelschicht, umfassend ein Etherlipid und ein Kopfgruppen-derivatisiertes Lipid, wobei das Etherlipid die chemische Formel: 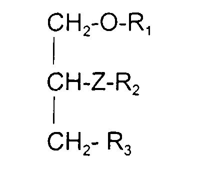
hat, wobei R₁ gleich Y₁Y₂ ist, Y₂ gleich CH₃ oder CO₂H ist und Y₁ gleich (CH₂)ₙ₁(CH=CH)ₙ₂(CH₂)ₙ₃(CH=CH)ₙ₄(CH₂)ₙ₅(CH=CH)ₙ₆(CH₂)ₙ₇(CH=CH)ₙ₈(CH₂)ₙ₉ ist,
wobei die Summe aus n1+2n2+n3+2n4+n5+2n6+n7+2n8+n9 gleich eine ganze Zahl von 3 bis 23 ist, n1 gleich null oder eine ganze Zahl von 3 bis 23 ist, n3 gleich null oder eine ganze Zahl von 1 bis 21 ist, n5 gleich null oder eine ganze Zahl von 1 bis 18 ist, n7 gleich null oder eine ganze Zahl von 1 bis 15 ist, n9 gleich null oder eine ganze Zahl von 1 bis 12 ist, und n2, n4, n6 und n8 unabhängig voneinander jeweils gleich null oder 1 sind,
wobei Z gleich O oder S ist,
wobei R₂ gleich Y₁Y₂ oder (C(X₁)ₙ₁₀(X₂)ₙ₁₁)ₙ₁₂CX₃X₄X₅ ist,
wobei X₁, X₂, X₃, X₄ und X₅ unabhängig voneinander jeweils H oder F sind, n10 gleich null, 1 oder 2 ist, n11 gleich null, 1 oder 2 ist, n12 gleich eine ganze Zahl von 1 bis 23 ist, und die Summe aus n10+n11 gleich 2 ist,
wobei R₃ die Formel R₅-P(O)₂-O-R₆ hat, R₅ gleich O, S oder NH ist, R₆ gleich CH₂CH₂N(CH₃)₃, CH₂CH₂NH₂, CH₂CH(OH)CH₂OH oder CH₂CH₂NHC(O)R₇ ist, und wobei R₇ Y₂CH₃ oder Y₂CO₂H ist.

2. Liposom nach Anspruch 1, wobei das Liposom einen Durchmesser von kleiner als etwa 200 nm hat.

3. Liposom nach Anspruch 2, wobei das Liposom einen Durchmesser von größer als etwa 50 nm bis kleiner als etwa 200 nm hat

4. Liposom nach Anspruch 1, wobei Y₂ gleich CH₃ ist.

5. Liposom nach Anspruch 4, wobei R₁ gleich (CH₂)ₙ₁CH₃ ist.

6. Liposom nach Anspruch 5, wobei R₁ gleich (CH₂)₁₇CH₃ ist.

7. Liposom nach Anspruch 1, wobei Z gleich O ist.

8. Liposom nach Anspruch 1, wobei R₂ gleich (C(X₁)ₙ₁₀(X₂)ₙ₁₁)ₙ₁₂CX₃X₄X₅ ist.

9. Liposom nach Anspruch 8, wobei n12 gleich eins ist.

10. Liposom nach Anspruch 9, wobei n12 gleich eins ist und R₂ gleich CH₂CH₃, CH₂CF₃ oder CF₂CF₃ ist.

11. Liposom nach Anspruch 1, wobei R₃ gleich O-P(O)₂-O-R₆ ist.

12. Liposom nach Anspruch 11, wobei R₃ gleich O-P(O)₂-O-CH₂CH₂N(CH₃)₃ ist.

13. Liposom mit einer Lipid-Doppelschicht, umfassend ein Etherlipid und ein Kopfgruppen-derivatisiertes Lipid, wobei das Etherlipid wie in Anspruch 1 definiert ist, mit der Maßgabe, daß n12 gleich null oder eine ganze Zahl von 1 bis 23 ist, wobei das Kopfgruppen-derivatisierte Lipid eine Phosphatidylethanolamin-Dicarbonsäure, bevorzugt Dioleoylphosphatidylethanolamin-Glutarsäure ist.

14. Liposom nach Anspruch 13, wobei das Phosphatidylethanolamin Dipalmitoylphosphatidylethanolamin, Palmitoyloleoylphosphatidylethanolamin oder Dioleoylphosphatidylethanolamin, bevorzugt Dioleoylphosphatidylethanolamin ist.

15. Liposom nach Anspruch 13, wobei die Dicarbonsäure Glutar-, Sebacin-, Succin-oder Weinsäure, bevorzugt Glutarsäure ist.

16. Liposom mit einer Lipid-Doppelschicht, umfassend ein Etherlipid und ein Kopfgruppen-derivatisiertes Lipid, wobei das Etherlipid wie in Anspruch 1 definiert ist, mit der Maßgabe, daß n12 gleich null oder eine ganze Zahl von 1 bis 23 ist, wobei das Kopfgruppen-derivatisierte Lipid ein Zirkulations-förderndes Lipid ist.

17. Liposom nach Anspruch 1, wobei die Doppelschicht ein Sterol umfaßt.

18. Liposom mit einer Lipid-Doppelschicht, umfassend ein Etherlipid und ein Kopfgruppen-derivatisiertes Lipid, wobei das Etherlipid wie in Anspruch 1 definiert ist, mit der Maßgabe, daß n12 gleich null oder eine ganze Zahl von 1 bis 23 ist, wobei die Doppelschicht ein neutrales Lipid umfaßt.

19. Liposom nach Anspruch 18, wobei das neutrale Lipid ein Phosphatidylcholin ist.

20. Liposom nach Anspruch 19, wobei das Phosphatidylcholin Ei-Phosphatidylcholin, Palmitoyloleoylphosphatidylcholin oder Dioleoylphosphatidylcholin, bevorzugt Dioleoylphosphatidylcholin ist.

21. Liposom mit einer Lipid-Doppelschicht, umfassend ein Etherlipid und ein Kopfgruppen-derivatisiertes Lipid, wobei das Etherlipid wie in Anspruch 1 definiert ist, mit der Maßgabe, daß n12 gleich null oder eine ganze Zahl von 1 bis 23 ist, umfassend ein Zirkulations-förderndes Lipid.

22. Liposom nach Anspruch 21, weiterhin umfassend ein Sterol und ein neutrales Lipid.

23. Liposom nach Anspruch 16, 18 oder 21, wobei das Etherlipid: 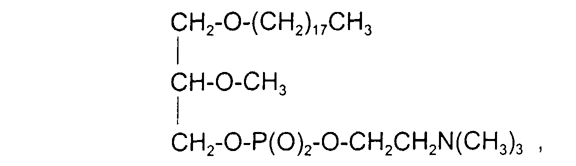 ist.

24. Liposom nach Anspruch 23, wobei das Kopfgruppen-derivatisierte Lipid Dioleoylphosphatidylethanolamin-Glutarsäure ist.

25. Liposom nach Anspruch 17 oder 22, wobei das Sterol Cholesterol ist.

26. Liposom nach Anspruch 22, wobei das neutrale Lipid Dioleoylphosphatidylcholin ist.

27. Liposom nach Anspruch 22, wobei die Doppelschicht etwa 20 Molprozent des Etherlipids, etwa 10 Molprozent Dioleoylphosphatidylethanolamin-Glutarsäure, etwa 30 Molprozent Cholesterol und etwa 40 Molprozent Dioleoylphosphatidylcholin umfaßt.

28. Liposom nach einem der Ansprüche 1 bis 27, umfassend ein zusätzliches biologisch aktives Mittel.

29. Pharmazeutische Zusammensetzung, umfassend einen pharmazeutisch akzeptablen Träger und das Liposom nach einem der Ansprüche 1 bis 28.

30. Verfahren zur Herstellung einer pharmazeutischen Zusammensetzung zur Verabreichung eines Etherlipids einem Lebewesen, umfassend einen pharmazeutisch akzeptablen Träger und ein Liposom mit einer Lipid-Doppelschicht, umfassend das Etherlipid und ein Kopfgruppen-derivatisiertes Lipid, wobei das Etherlipid die chemische Formel: 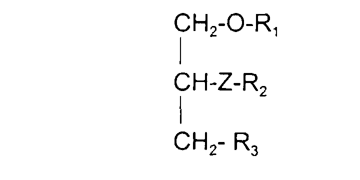
hat, wobei R₁ gleich Y₁Y₂ ist, Y₂ gleich CH₃ oder CO₂H ist und Y₁ gleich (CH₂)ₙ₁(CH=CH)ₙ₂(CH₂)ₙ₃(CH=CH)ₙ₄(CH₂)ₙ₅(CH=CH)ₙ₆(CH₂)ₙ₇(CH=CH)ₙ₈(CH₂)ₙ₉ ist,
wobei die Summe aus n1+2n2+n3+2n4+n5+2n6+n7+2n8+n9 gleich eine ganze Zahl von 3 bis 23 ist, n1 gleich null oder eine ganze Zahl von 3 bis 23 ist, n3 gleich null oder eine ganze Zahl von 1 bis 21 ist, n5 gleich null oder eine ganze Zahl von 1 bis 18 ist, n7 gleich null oder eine ganze Zahl von 1 bis 15 ist, n9 gleich null oder eine ganze Zahl von 1 bis 12 ist, und n2, n4, n6 und n8 unabhängig voneinander jeweils gleich null oder 1 sind,
wobei Z gleich O oder S ist,
wobei R₂ gleich Y₁Y₂ oder (C(X₁)ₙ₁₀(X₂)ₙ₁₁)ₙ₁₂CX₃X₄X₅ ist,
wobei X₁, X₂, X₃, X₄ und X₅ unabhängig voneinander jeweils H oder F sind, n10 gleich null, 1 oder 2 ist, n11 gleich null, 1 oder 2 ist, n12 gleich eine ganze Zahl von 1 bis 23 ist, und die Summe aus n10+n11 gleich 2 ist,
und wobei R₃ die Formel R₅-P(O)₂-O-R₆ hat, R₅ gleich O, S oder NH ist, R₆ gleich CH₂CH₂N(CH₃)₃, CH₂CH₂NH₂, CH₂CH(OH)CH₂OH oder CH₂CH₂NHC(O)R₇ ist, und R₇ Y₂CH₃ oder Y₂CO₂H ist.

31. Verfahren nach Anspruch 30, wobei das Lebewesen ein Mensch ist.

32. Verfahren nach Anspruch 30, wobei das Lebewesen an einem Krebs erkrankt ist und wobei die pharmazeutische Zusammensetzung eine gegen Krebs wirksame Menge des Etherlipids umfaßt.

33. Verfahren nach Anspruch 32, wobei der Krebs ein Lungen-, Hirn-, Darm-, Eierstock- oder Brustkrebs ist.

34. Verfahren nach Anspruch 32, wobei die gegen Krebs wirksame Menge des Etherlipids von etwa 0,1 mg des Etherlipids pro kg Körpergewicht des Lebewesens, dem die pharmazeutische Zusammensetzung verabreicht wird, bis etwa 1000 mg des Etherlipids pro kg Körpergewicht beträgt.

35. Verfahren nach Anspruch 30, wobei das Lebewesen von einer Entzündungserkrankung befallen ist und die pharmazeutische Präparation eine gegen Entzündung wirksame Menge des Etherlipids umfaßt.

36. Verfahren nach Anspruch 35, wobei die Entzündungserkrankung ein Arthritisleiden, eine Asthmaerkrankung oder eine Allergie ist.

37. Verfahren nach Anspruch 35, wobei die gegen Entzündung wirksame Menge des Etherlipids von etwa 0,1 mg des Etherlipids pro kg Körpergewicht des Lebewesens, dem die pharmazeutische Zusammensetzung verabreicht wird, bis etwa 1000 mg des Etherlipids pro kg Körpergewicht beträgt.

38. Verfahren nach Anspruch 30, wobei die pharmazeutische Zusammensetzung ein zusätzliches biologisch aktives Mittel umfaßt.

39. Verfahren nach Anspruch 38, wobei das zusätzliche biologisch aktive Mittel ein antineoplastisches Mittel, ein antimikrobielles Mittel, ein entzündungshemmendes Mittel, ein therapeutisches Lipid oder ein das Wachstum von hämopoetischen Zellen stimulierendes Mittel ist.

40. Verfahren nach Anspruch 30, wobei das Liposom einen Durchmesser von größer als etwa 50 nm bis kleiner als etwa 200 nm hat.

41. Verfahren nach Anspruch 30, wobei R₁ gleich (CH₂)₁₇CH₃ ist.

42. Verfahren nach Anspruch 30, wobei R₂ gleich CH₃ ist.

43. Verfahren nach Anspruch 30, wobei R₃ gleich O-P(O)₂-O-CH₂CH₂N(CH₃)₃ ist.

44. Verfahren zur Herstellung einer pharmazeutischen Zusammensetzung zur Verabreichung eines Etherlipids einem Lebewesen, umfassend einen pharmazeutisch akzeptablen Träger und ein Liposom mit einer Lipid-Doppelschicht, umfassend das Etherlipid und ein Kopfgruppen-derivatisiertes Lipid, wobei das Etherlipid wie in Anspruch 1 definiert ist, mit der Maßgabe, daß n12 gleich null oder eine ganze Zahl von 1 bis 23 ist, wobei das Kopfgruppen-derivatisierte Lipid eine Phosphatidylethanolamin-Dicarbonsäure, bevorzugt Dioleoylphosphatidylethanolamin-Glutarsäure ist.

45. Verfahren zur Herstellung einer pharmazeutischen Zusammensetzung zur Verabreichung eines Etherlipids einem Lebewesen, umfassend einen pharmazeutisch akzeptablen Träger und ein Liposom mit einer Lipid-Doppelschicht, umfassend das Etherlipid und ein Kopfgruppen-derivatisiertes Lipid, wobei das Etherlipid wie in Anspruch 1 definiert ist, mit der Maßgabe, daß n12 gleich null oder eine ganze Zahl von 1 bis 23 ist, wobei die Doppelschicht weiterhin ein Sterol und ein neutrales Lipid umfaßt.

46. Verfahren nach Anspruch 45, wobei das Kopfgruppen-derivatisierte Lipid Dioleoylphosphatidylethanolamin-Glutarsäure ist, das Sterol Cholesterol ist, das neutrale Lipid Dioleoylphosphatidylcholin ist, und das Etherlipid: 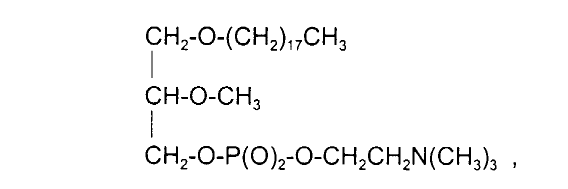 ist.

47. Verfahren nach Anspruch 46, wobei die Doppelschicht etwa 20 Molprozent des Etherlipids, etwa 10 Molprozent Dioleoylphosphatidylethanolamin-Glutarsäure, etwa 30 Molprozent Cholesterol und etwa 40 Molprozent Dioleoylphosphatidylcholin umfaßt.

48. Verfahren zur Herstellung einer pharmazeutischen Zusammensetzung zur Verabreichung eines Etherlipids einem Lebewesen, umfassend einen pharmazeutisch akzeptablen Träger und ein Liposom mit einer Lipid-Doppelschicht, umfassend das Etherlipid und ein Kopfgruppen-derivatisiertes Lipid, wobei das Etherlipid wie in Anspruch 1 definiert ist, mit der Maßgabe, daß n12 gleich null oder eine ganze Zahl von 1 bis 23 ist, wobei das Liposom ein Zirkulations-förderndes Lipid umfaßt.

## Revendications

1. Liposome comportant une bicouche lipidique qui comprend un lipide-éther et un lipide transformé en dérivé sur le groupe de tête, le lipide-éther correspondant à la formule suivante: 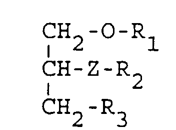
dans laquelle R₁ est Y₁Y₂, Y₂ est CH₃ ou CO₂H et Y₁ est (CH₂)ₙ₁(CH=CH)ₙ₂(CH₂)ₙ₃(CH=CH)ₙ₄(CH₂)ₙ₅(CH=CH)ₙ₆ (CH₂)ₙ₇-(CH=CH)ₙ₈(CH₂)ₙ₉,
la somme de n1 + 2n2 + n3 + 2n4 + n5 + 2n6 + n7 + 2n8 + n9 étant égale à un nombre entier allant de 3 à 23, n1 est égal à zéro ou est un nombre entier allant de 3 à 23; n3 est égal à zéro ou est un nombre entier allant de 1 à 21;
n5 est égal à zéro ou est un nombre entier allant de 1 à 18; n7 est égal à zéro ou est un nombre entier allant de 1 à 15; n9 est égal à zéro ou est un nombre entier allant de 1 à 12; et n2, n4, n6 et n8 représentent chacun, indépendamment, zéro ou 1;Z est O ou S;
R₂ est Y₁Y₂ ou (C(X₁)ₙ₁₀(X₂)n₁₁)ₙ₁₂CX₃X₄X₅;
chacun des symboles X₁, X₂, X₃, X₄ et X₅ est indépendamment H ou F, n10 est zéro, 1 ou 2, nll est zéro, 1 ou 2, n12 est un nombre entier allant de 1 à 23, et la somme de n10 + n11 est égale à 2;
R₃ correspond à la formule R₅-P(O)₂-O-R₆, R₅ est O, S ou NH, R₆ est CH₂CH₂N(CH₃)₃, CH₂CH₂NH₂, CH₂CH(OH)CH₂OH ou CH₂CH₂NHC(O)R₇, et R₇ est Y₂CH₃ ou Y₂CO₂H.

2. Liposome de la revendication 1, le liposome ayant un diamètre inférieur à environ 200 nm.

3. Liposome de la revendication 2, le liposome ayant un diamètre de plus d'environ 50 nm à moins d'environ 200 nm.

4. Liposome de la revendication 1, dans lequel Y₂ est CH₃.

5. Liposome de la revendication 4, dans lequel R₁ est (CH₂)ₙ₁CH₃.

6. Liposome de la revendication 5, dans lequel R₁ est (CH₂)₁₇CH₃.

7. Liposome de la revendication 1, dans lequel Z est O.

8. Liposome de la revendication 1, dans lequel R₂ est (C(X₁)ₙ₁₀(X₂)ₙ₁₁)ₙ₁₂CX₃X₄X₅.

9. Liposome de la revendication 8, dans lequel n12 est égal à 1.

10. Liposome de la revendication 9, dans lequel n12 est égal à 1 et R₂ est CH₂CH₃, CH₂CF₃ ou CF₂CF₃.

11. Liposome de la revendication 1, dans lequel R₃ est O-P(O)₂-O-R₆.

12. Liposome de la revendication 11, dans lequel R₃ est -O-P(O)₂-O-CH₂CH₂N(CH₃)₃.

13. Liposome comportant une bicouche lipidique comprenant un lipide-éther et un lipide transformé en dérivé sur le groupe de tête, dans lequel le lipide-éther est défini comme dans la revendication 1, étant entendu que n12 est égal à zéro ou est un nombre entier allant de 1 à 23, le lipide transformé en dérivé sur le groupe de tête étant de type phosphatidyléthanolamine-acide dicarboxylique, de préférence le dioléoylphosphatidyléthanolamine-acide glutarique.

14. Liposome de la revendication 13, dans lequel la phosphatidyléthanolamine est la dipalmitoylphosphatidyléthanolamine, la palmitoyloléoylphosphatidyléthanolamine ou la dioléoylphosphatidyléthanolamine, de préférence la dioléoylphosphatidyléthanolamine.

15. Liposome de la revendication 13, dans lequel l'acide dicarboxylique est l'acide glutarique, sébacique, succinique ou tartrique, de préférence l'acide glutarique.

16. Liposome comportant une bicouche lipidique comprenant un lipide-éther et un lipide transformé en dérivé sur le groupe de tête, dans lequel le lipide-éther est défini comme dans la revendication 1, étant entendu que n12 est égal à zéro ou est un nombre entier allant de 1 à 23 et dans lequel le lipide transformé en dérivé sur le groupe de tête est un lipide stimulant la circulation.

17. Liposome de la revendication 1, dans lequel la bicouche comprend un stérol.

18. Liposome comportant une bicouche lipidique comprenant un lipide-éther et un lipide transformé en dérivé sur le groupe de tête, dans lequel le lipide-éther est défini comme dans la revendication 1, étant entendu que n12 est égal à zéro ou est un nombre entier allant de 1 à 23, et dans lequel la bicouche comprend un lipide neutre.

19. Liposome de la revendication 18, dans lequel le lipide neutre est une phosphatidylcholine.

20. Liposome de la revendication 19, dans lequel la phosphatidylcholine est la phosphatidylcholine d'oeuf, la palmitoyloléoylphosphatidylcholine ou la dioléoylphosphatidylcholine, de préférence le dioléoylphosphatidylcholine.

21. Liposome comportant une bicouche lipidique comprenant un lipide-éther et un lipide transformé en dérivé sur le groupe de tête, dans lequel le lipide-éther est défini comme dans la revendication 1, étant entendu que n12 est égal à zéro ou est un nombre entier allant de 1 à 23, comprenant un lipide stimulant la circulation.

22. Liposome de la revendication 21, comprenant en outre un stérol et un lipide neutre.

23. Liposome de la revendication 16, 18 ou 21, dans lequel le lipide-éther est

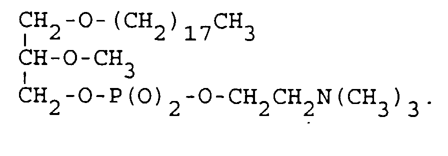

24. Liposome de la revendication 23, dans lequel le lipide transformé en dérivé sur le groupe de tête est le dioléoylphosphatidyléthanolamine-acide glutarique.

25. Liposome de la revendication 17 ou 22, dans lequel le stérol est le cholestérol.

26. Liposome de la revendication 22, dans lequel le lipide neutre est la dioléoylphosphatidylcholine.

27. Liposome de la revendication 22, dans lequel la bicouche comprend environ 20 % en moles du lipide-éther, environ 10 % en moles de dioléoylphosphatidyléthanolamine-acide glutarique, environ 30 % en moles de cholestérol et environ 40 % en moles de dioléoylphosphatidylcholine.

28. Liposome de l'une quelconque des revendications 1 à 27, comprenant un agent bioactif supplémentaire.

29. Composition pharmaceutique, comprenant un véhicule pharmaceutiquement acceptable et le liposome de l'une quelconque des revendications 1 à 28.

30. Procédé de préparation d'une composition pharmaceutique pour administration d'un lipide-éther à un animal, comprenant un véhicule pharmaceutiquement acceptable et un liposome comportant une bicouche lipidique comprenant le lipide-éther et un lipide transformé en dérivé sur le groupe de tête, le lipide-éther correspondant à la formule suivante: 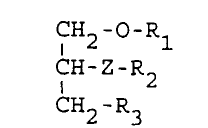
dans laquelle R₁ est Y₁Y₂, Y₂ est CH₃ ou CO₂H et Y₁ est (CH₂)ₙ₁(CH=CH)ₙ₂(CH₂)ₙ₃(CH=CH)ₙ₄(CH₂)ₙ₅(CH=CH)ₙ₆(CH₂)ₙ₇-(CH=CH)n₈(CH₂)n₉,
la somme de n1 + 2n2 + n3 + 2n4 + n5 + 2n6 + n7 + 2n8 + n9 étant égale à un nombre entier allant de 3 à 23, n1 est égal à zéro ou est un nombre entier allant de 3 à 23; n3 est égal à zéro ou est un nombre entier allant de 1 à 21;
n5 est égal à zéro ou est un nombre entier allant de 1 à 18; n7 est égal à zéro ou est un nombre entier allant de 1 à 15; n9 est égal à zéro ou est un nombre entier allant de 1 à 12; et n2, n4, n6 et n8 représentent chacun, indépendamment, zéro ou 1;
Z est O ou S;
R₂ est Y₁Y₂ ou (C(X₁)n₁₀(X₂)n₁₁)n₁₂CX₃X₄X₅;
chacun des symboles X₁, X₂, X₃, X₄ et X₅ est indépendamment H ou F, n10 est zéro, 1 ou 2, n11 est zéro, 1 ou 2, n12 est un nombre entier allant de 1 à 23, et la somme de n10 + nll est égale à 2;
R₃ correspond à la formule R₅-P(O)₂-O-R₆, R₅ est O, S ou NH, R₆ est CH₂CH₂N(CH₃)₃, CH₂CH₂NH₂, CH₂CH(OH)CH₂OH ou CH₂CH₂NHC(O)R₇, et R₇ est Y₂CH₃ ou Y₂CO₂H.

31. Procédé de la revendication 30, dans lequel l'animal est un sujet humain.

32. Procédé de la revendication 30, dans lequel l'animal est atteint d'un cancer et dans lequel la préparation pharmaceutique comprend une quantité anticancéreuse efficace du lipide-éther.

33. Procédé de la revendication 32, dans lequel le cancer est un cancer du poumon, du cerveau, du côlon, de l'ovaire ou du sein.

34. Procédé de la revendication 32, dans lequel la quantité anticancéreuse efficace du lipide-éther va d'environ 0,1 mg du lipide-éther par kg du poids corporel de l'animal auquel est administrée la composition pharmaceutique, à environ 1 000 mg du lipide-éther par kg de poids corporel.

35. Procédé de la revendication 30, dans lequel l'animal est atteint d'un trouble inflammatoire, et la composition pharmaceutique comprend une quantité anti-inflammatoire efficace du lipide-éther.

36. Procédé de la revendication 35, dans lequel le trouble inflammatoire est un état arthritique, un trouble asthmatique ou une réaction allergique;

37. Procédé de la revendication 35, dans lequel la quantité anti-inflammatoire efficace du lipide-éther va d'environ 0,1 mg du lipide-éther par kg du poids corporel de l'animal auquel est administrée la composition pharmaceutique, à environ 1 000 mg du lipide-éther par kg de poids corporel.

38. Procédé de la revendication 30, dans lequel la composition pharmaceutique comprend un agent biologiquement actif supplémentaire.

39. Procédé de la revendication 38, dans lequel l'agent biologiquement actif supplémentaire est un agent antinéoplasique, un agent antimicrobien, un agent anti-inflammatoire, un lipide thérapeutique ou un agent stimulant la croissance de cellules hématopoïétiques.

40. Procédé de la revendication 30, dans lequel le liposome a un diamètre de plus d'environ 50 nm à moins d'environ 200 nm.

41. Procédé de la revendication 30, dans lequel R₁ est (CH₂)₁₇CH₃.

42. Procédé de la revendication 30, dans lequel R₂ est CH₃.

43. Procédé de la revendication 30, dans lequel R₃ est -O-P(O)₂-O-CH₂CH₂N(CH₃)₃.

44. Procédé de préparation d'une composition pharmaceutique pour administration d'un lipide-éther à un animal, comprenant un véhicule pharmaceutiquement acceptable et un liposome comportant une bicouche lipidique comprenant le lipide-éther et un lipide transformé en dérivé sur le groupe de tête, dans lequel le lipide-éther est défini comme dans la revendication 1, étant entendu que n12 est égal à zéro ou est un nombre entier allant de 1 à 23, et dans lequel le lipide transformé en dérivé sur le groupe de tête est de type phosphatidyléthanolamine-acide dicarboxylique, de préférence le dioléoylphosphatidyléthanolamine-acide glutarique.

45. Procédé de préparation d'une composition pharmaceutique pour administration d'un lipide-éther à un animal, comprenant un véhicule pharmaceutiquement acceptable et un liposome comportant une bicouche lipidique comprenant le lipide-éther et un lipide transformé en dérivé sur le groupe de tête, dans lequel le lipide-éther est défini comme dans la revendication 1, étant entendu que n12 est égal à zéro ou est un nombre entier allant de 1 à 23, et dans lequel la bicouche comprend en outre un stérol et un lipide neutre.

46. Procédé de la revendication 45, dans lequel le lipide transformé en dérivé sur le groupe de tête est le dioléoylphosphatidyléthanolamine-acide glutarique, le stérol est le cholestérol, le lipide neutre est la dioléoylphosphatidylcholine et le lipide-éther est:

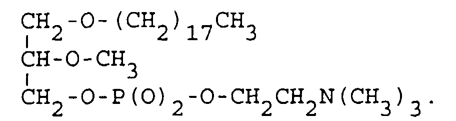

47. Procédé de la revendication 46, dans lequel la bicouche comprend environ 20 % en moles du lipide-éther, environ 10 % en moles de dioléoylphosphatidyléthanolamine-acide glutarique, environ 30 % en moles de cholestérol et environ 40 % en moles de dioléoylphosphatidylcholine.

48. Procédé de préparation d'une composition pharmaceutique pour l'administration d'un lipide-éther à un animal, comprenant un véhicule pharmaceutiquement acceptable et un liposome comportant une bicouche lipidique comprenant le lipide-éther et un lipide transformé en dérivé sur le groupe de tête, dans lequel le lipide-éther est défini comme dans la revendication 1, étant entendu que n12 est égal à zéro ou est un nombre entier allant de 1 à 23, et dans lequel le liposome comprend un lipide stimulant la circulation.
